# EUROPÄISCHE PATENTSCHRIFT

(11) **EP 0 876 807 B1**
(45) Veröffentlichungstag und Bekanntmachung des Hinweises auf die Patenterteilung: **27.10.1999**
(21) Anmeldenummer: 98107940.3
(22) Anmeldetag: 30.04.1998
(51) Int. Cl.: A61F 2/76

(54) **Kombination eines Schaftadapters zum Verbinden einer Stumpffassung mit einem Prothesenschaft und eines mit der Stumpffassung verbundenen Stiftes**
Combination of an adaptive shaft interconnector between a stump harness and the body of a prosthesis and a pin connected to the stump harness
Combinaison d'un adaptateur pour tige pour connecter une emplanture de moignon avec la tige d'une prothèse et d'une broche jointe à la tige de prothèse

(30) Priorität: 05.05.1997 DE 19718580
(43) Veröffentlichungstag der Anmeldung: 11.11.1998
(73) Patentinhaber: BIEDERMANN MOTECH GmbH, 78054 Villingen-Schwenningen (DE)
(72) Erfinder: Biedermann, Lutz, 78048 VS-Villingen (DE); Matthis, Wilfried, 79367 Weisweil (DE); Piro, Markus, 78078 Niedereschach (DE)
(74) Vertreter: Prüfer, Lutz H., Dipl.-Phys.

(56) Entgegenhaltungen:
- FR-A- 2 091 619
- GB-A- 2 162 069
- US-A- 3 422 462
- US-A- 5 116 382
- US-A- 5 376 129
- US-A- 5 507 837

## Beschreibung

Die Erfindung betrifft einen Schaftadapter zum Verbinden einer Stumpffassung mit einem Prothesenschaft und eines mit der Stumpffasung verbundenen Stiftes nach dem Oberbegriff des Anspruchs 1.

Eine Stumpffassung ist beispielsweise aus der US-PS 5,507,334 bekannt. Diese ist als Silikonstrumpf ausgebildet, den der Träger über den mit der Prothese zu verbindenden Stumpf zieht. An dem mit dem Stumpf zu verbindenden Ende weist der Strumpf einen als Schraube ausgebildeten Stift auf, der in einen Adapterteil eines Prothesenschaftes eingeschraubt wird. Der Proband achtet dabei darauf, daß der Strumpf so angezogen wird, daß der Stift in der neutralen nicht geneigten Position des Stumpfes sich in vertikaler Richtung erstreckt und somit in eine entsprechende Bohrung des Schaftes einführbar ist. Dabei tritt das Problem auf, daß der Stift zwar sich in vertikaler Richtung erstreckt, sich in vielen Fällen aber wegen der unterschiedlichen Ausbildung der Beine, beispielsweise als X-Beine, nicht unter dem eigentlichen Tragpunkt des Stumpfes liegt. Dadurch entstehen für den Probanden Schwierigkeiten durch eine erhebliche Belastung.

Aus der GB 2 162 069 A und der US 3,422,462 ist eine Kombination nach dem Oberbegriff des Anspruchs 1 bekannt.

Aufgabe der Erfindung ist es, eine Kombination der eingangs beschriebenen Art zu schaffen, mit dem eine Verbindung einer Stumpffassung mit einem Prothesenschaft derart verbessert werden kann, daß die Trageigenschaften wesentlich verbessert werden, und mit dem schnell eine sichere Verbindung eines den Stumpf aufnehmenden Strumpfes mit der Strumpffassung möglich ist.

Diese Aufgabe wird durch die in dem Patentanspruch 1 gekennzeichneten Kombination gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren. Von den Figuren zeigen:
- Fig. 1: eine Seitenansicht einer mit einem Schaftadapter mit einem Prothesenschaft verbundenen Stumpffassung im Schnitt;
- Fig. 2: den Schaftadapter in explosionsartiger Darstellung;
- Fig. 3: eine abgewandelte Ausführungsform in explosionsartiger Darstellung;
- Fig. 4: eine vergrößerte Detaildarstellung aus der ersten Ausführungsform in Arretierstellung;
- Fig. 5: dasselbe Detail in gelöstem Zustand;
- Fig. 6: eine explosionsartige Darstellung einer abgewandelten Ausführungsform; und
- Fig. 7: ein Detail der Vorrichtung.

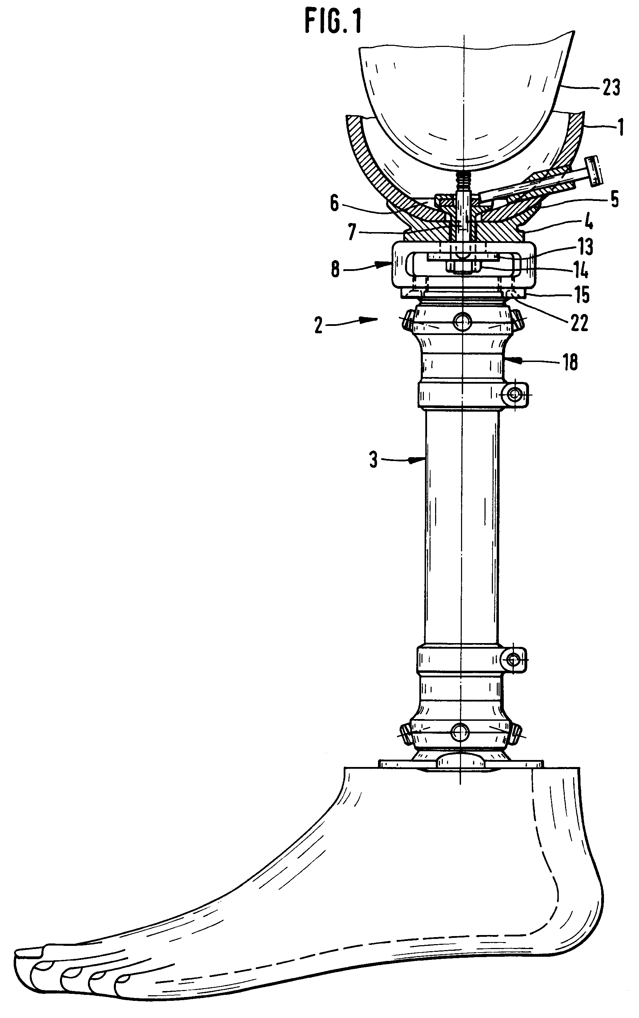

Wie aus Fig. 1 ersichtlich ist, ist eine Stumpffassung 1 mittels einer Adaptereinrichtung 2 mit einem Prothesenschaft 3 verbunden.

Die Adaptereinrichtung 2 umfaßt einen Schaftansatz 4, der auf seiner der Stumpffassung 1 zugewandten Seite einen sphärischen Bereich 5 zur Aufnahme der Stumpffassung 1 aufweist. Auf der Stumpffassung ist innen eine zu dem sphärischen Bereich 5 passend ausgebildete sphärische Klemmscheibe 6 vorgesehen, die dazu dient, zusammen mit dem sphärischen Bereich 5 den Strumpf einzuklemmen. Auf seiner dem sphärischen Bereich 5 abgewandten Seite ist der Schaftansatz 4 plan ausgebildet. Die Klemmscheibe 6 und der Schaftansatz 4 weisen eine Mittenbohrung zur Aufnahme einer als Hohlstift 7 dienenden Schraube auf. Auf der dem sphärischen Bereich 5 abgewandten Seite grenzt an den Schaftansatz 4 ein als Zwischenstück ausgebildetes Adapterelement 8. Dieses ist als ein Art Käfig aufgebaut mit einer Deckwand 9, Seitenwänden 10 und einer Bodenwand 11. Die Deckwand 9 weist eine Ausnehmung 12 auf, deren Abmessung wesentlich größer als der Durchmesser des Hohlstiftes 7 ist. Auf der der Bodenwand 11 zugewandten Seite der Deckwand 9 ist eine Unterlegscheibe 13 vorgesehen, deren Außenabmessungen größer sind als die Innenabmessungen der Ausnehmung 12. Auf der der Deckwand 9 abgewandten Seite der Unterlegscheibe 13 ist eine Mutter 14 zum Aufschrauben auf den schraubenförmigen Hohlstift 7 angeordnet. Die Seitenwände 10 sind so bemessen, daß der Abstand der Bodenwand 11 von der Deckwand 9 größer ist als die in axialer Richtung gesehene Länge des aufzunehmenden Teiles des Hohlstiftstiftes 7 einschließlich Unterlegscheibe und Mutter. Wenigstens eine der Seitenwände weist, wie aus den Figuren ersichtlich ist, eine Ausnehmung auf, die es ermöglicht, die Mutter von außen mittels eines Schraubenschlüssels festzuziehen bzw. zu lösen.

Auf der der Deckwand 9 abgewandten Seite der Bodenwand 11 ist ein weiterer auf der zugewandten Seite eben ausgebildeter Schaftadapter 15 vorgesehen, welcher auf seiner dem Zwischenstück abgewandten Seite eine im wesentlichen sphärisch ausgebildete Fläche 16 und einen koaxial angeordneten pyramidenstumpfförmigen Abschnitt 17 aufweist. Der Schaftadapter ist mit Schrauben 32 mit dem Adapterelement 8 verbunden. Der Pyramidenstumpf erstreckt sich in der Weise, daß die Basis der Pyramide auf der dem Zwischenstück abgewandten Seite liegt. Auf der den Pyramidenstumpf 17 aufweisenden Seite folgt ein als Klemmadapter ausgebildetes Element 18, welches auf seiner dem Pyramidenstumpf zugewandten Seite eine Ausnehmung mit einem der sphärischen Fläche 16 angepaßten sphärischen Wandung besitzt. Von außen greifen vier Setzschrauben 19 in die Ausnehmung ein, deren Achsen jeweils senkrecht zu den Wandungen des pyramidenstumpfförmigen Ansatzes gerichtet sind.

Auf der gegenüberliegenden Seite weist der Klemmadapter 18 eine Bohrung mit einer geschlitzten Wandung und einen Klemmring 20 auf. Die Bohrung ist wenig größer als der Außendurchmesser eines damit zu verbindenden Rohres 21 des Prothesenschaftes 3. Die Bemessung ist so gewählt, daß beim Anziehen des Klemmringes eine feste Verbindung zwischen dem Klemmelement 18 und dem Rohr 21 erfolgt.

Im Betrieb wird die Klemmscheibe 6 von innnen in die Stumpffassung 1 eingesetzt, dann wird die Stumpffassung 1 auf den sphärischen Bereich 5 des Schaftansatzes 4 aufgesetzt, und es wird die Schraube 7 durch die Bohrungen hindurchgeführt. Das als Zwischenstück ausgebildete Adapterelement 8 wird jetzt so relativ zu dem Schaftansatz 4 seitlich verschoben, daß die Mittenachse der Bodenwand 11 relativ zu Mittenachse des Stiftes 7 die gewünschte Position einnimmt. Dann wird durch Anziehen der Mutter 14 die Schraube in ihrer Position an der Deckwand 9 fixiert. Der Schaftadapter 15 und das Adapterelement 8 werden nun (oder sind auch bereits vorher) mittels angedeuteter Schrauben 22 so miteinander verbunden, daß die Mittenachse des Schaftadapters 15 und die Mittenachse der Bodenwand 11 zusammenfallen. Durch geeignetes Justieren mittels der Setzschrauben 19 kann eine winkelmäßige Einstellung zwischen dem Schaftadapter 15 und dem Element 18 vorgenommen werden. Dieses ist mittels des Klemmringes 20 fest mit dem Rohr 21 koaxial verbunden.

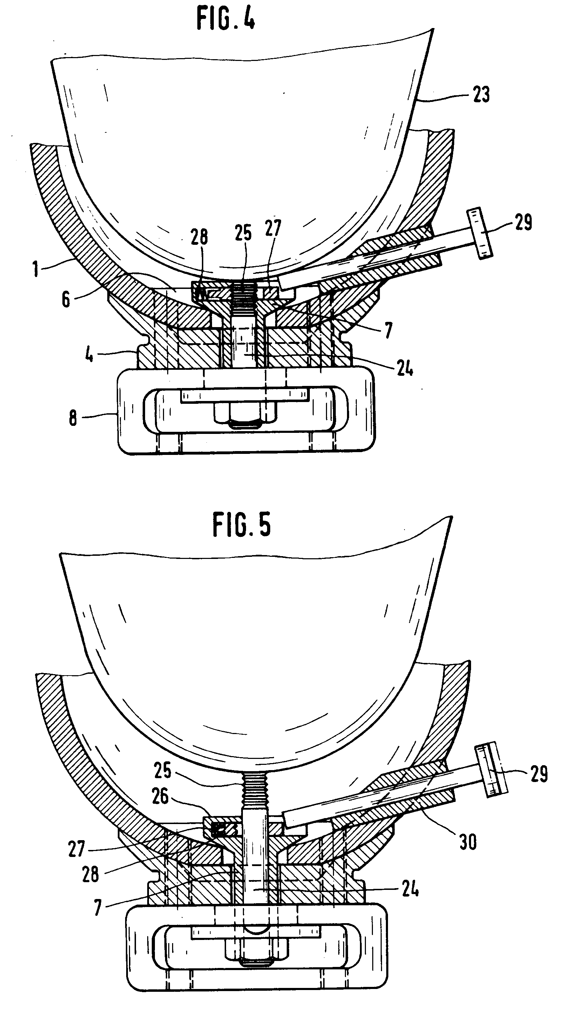

In die oben beschriebene Vorrichtung wird in der am besten aus den Figuren 4 und 5 ersichtlichen Weise ein einen Stumpf aufnehmender Strumpf 23 aufgenommen. Zu diesem Zweck weist der Strumpf 23 an seinem bodenseitigen Mittelpunkt einen Bolzen 24 auf. Der Bolzen hat einen Außendurchmesser, der im wesentlichen gleich dem Innendurchmesser der Innenbohrung des Hohlstiftes 7 ist, und der um so viel kleiner ist, daß er in der Innenbohrung gleiten kann. Der Bolzen 24 weist in dem gezeigten Ausführungsbeispiel unmittelbar an den Strumpf 23 angrenzend einen rillen- oder ratschenförmigen Abschnitt 25 auf. In dem Kopf 26 des Hohlstiftes 7 ist ein Verriegelungselement vorgesehen, welches eine sich senkrecht zur Symmetrieachse des Hohlstiftes 7 erstreckende Scheibe 27 auffaßt, und diese Scheibe ist durch eine Feder in Richtung zu der Symmetrieachse hin vorgespannt. Sie weist eine Bohrung auf, die größer als der Durchmesser des Bolzens 24 ist. Ferner ist auf der der Feder abgewandten Seite ein Druckstift 29 vorgesehen, der in einer Führung 30 so gelagert ist, daß er bei Krafteinwirkung der am besten aus Fig. 5 ersichtlichen Weise auf die Scheibe 27 einwirkt und diese gegen die Kraft der Feder 28 aus ihrer in Fig. 4 gezeigten vorgeschobenen Stellung in die in Fig. 5 gezeigte zurückgeschobene Stellung bewegt. Ohne Krafteinwirkung des Druckstiftes 29 verriegelt die Scheibe 27 in der in Fig. 4 gezeigten Weise durch Ineingriffbringen mit dem rillenförmigen Abschnitt 25 den Bolzen 24 in der eingeführten Stellung. Bei Einwirken einer Kraft auf den Druckstift 29 wird die Verriegelung freigegeben, und der Strumpf kann mit Bolzen aus dem Hohlstift 7 herausgenommen bzw. in diesen eingeführt werden.

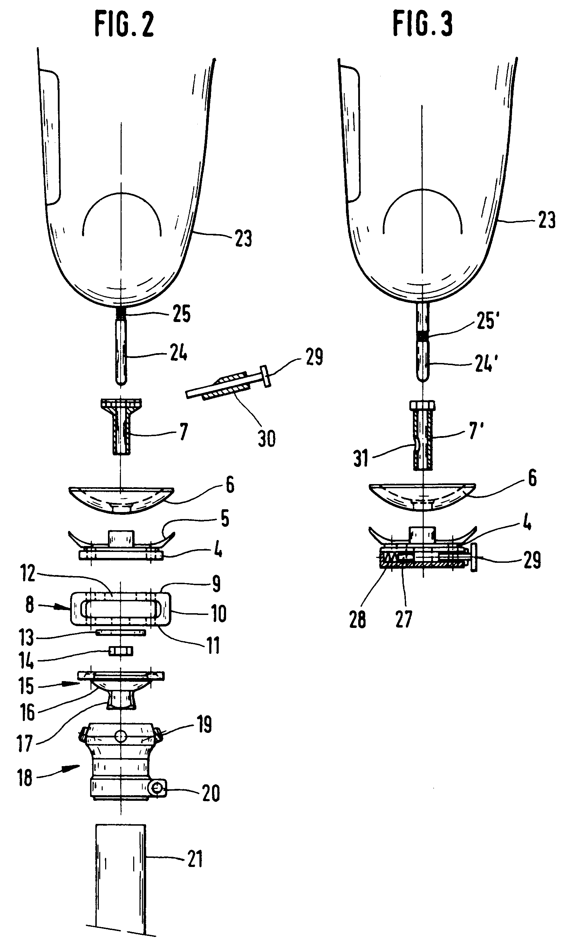

Die in Fig. 3 gezeigte Ausführungsform unterscheidet sich von der oben beschriebenen Ausführungsform lediglich dadurch, daß die Verriegelungseinrichtung nicht in dem Kopf des Hohlbolzens 7, sondern am Boden des Schaftansatzes 4 vorgesehen ist. Zu diesem Zweck ist bodenseitig von dem Schaftansatz 4 eine Kammer mit einem sich senkrecht zur längsseitigen Symmetrieachse der Vorrichtung erstreckender Schlitz vorgesehen, in dem wie in dem obigen Beispiel eine als Schieber ausgebildete Scheibe 27' vorgesehen ist, die mit einer Feder 28 in Richtung zur Mittenachse vorgespannt ist. Es ist wiederum ein auf die Scheibe einwirkender Druckstift 29 vorgesehen, mit dem die schieberförmige Scheibe 27 aus der davor gespannten Arretierstellung, die der Stellung in Fig. 4 entspricht, in die der Fig. 5 entsprechende gelöste Stellung zurückgeschoben werden kann. Der Hohlstift 7' unterscheidet sich von der ersten Ausführungsform dadurch, daß er eine Öffnung 31 aufweist, durch die die schieberförmige Scheibe 27 in das Innere greift und auf einen entsprechender Höhe angebrachten rillenförmigen Abschnitt 25' des Bolzens 24' zum Arretieren einwirkt.

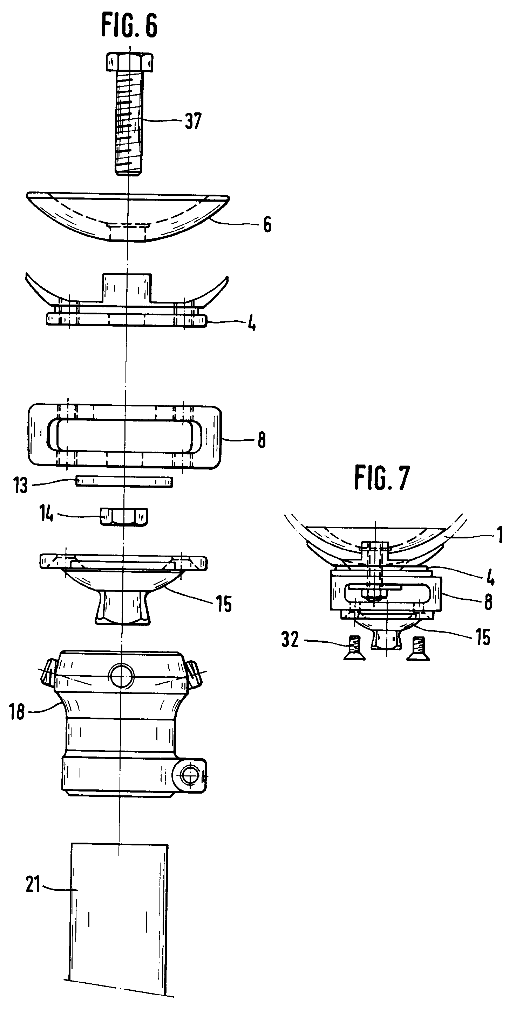

Die in Fig. 6 gezeigte Ausführungsform unterscheidet sich von den bisherigen Ausführungsformen lediglich dadurch, daß anstelle des zur Aufnahme des Bolzens 24 hohl ausgebildeten Stiftes 7 ein normaler Schraubbolzen 37 vorgesehen ist.

In Fig. 7 ist eine Stellung der zuvor beschriebenen Ausführungsformen gezeigt, bei der eine seitliche Versetzung zwischen Stumpffassung 1 und dem mit dem Prothesenschaft zu verbindenden Adapterelement 8 erfolgt ist.

## Patentansprüche

1. Kombination eines Schaftadapters zum Verbinden einer Stumpffassung mit einem Prothesenschaft und eines mit der Stumpffassung (1) verbundenen Stiftes (7, 7'), wobei der Schaftadapter ein Adapterelement (8) mit einer Ausnehmung (12) zur Aufnahme des Stiftes (7, 7') aufweist und wobei die Ausnehmung (12) in ihrem Querschnitt derart größer als die Querschnittsabmessung des Stiftes (7, 7') ist, daß der Stift (7, 7') in der Ausnehmung (12) verschiebbar ist, und wobei die Fixierung des Stiftes (7, 7') in jeder Position der Ausnehmung (12) möglich ist,
dadurch gekennzeichnet, daß der Stift (7) zur Aufnahme eines mit einem aufzunehmenden Strumpf (23) verbundenen Bolzens (24) als Hohlstift ausgebildet ist und eine Verriegelungseinrichtung (27, 28, 29) zum Verriegeln des Bolzens in einer vorgegebenen Stellung in dem Hohlstift (7) vorgesehen ist.

2. Kombination nach Anspruch 1, wobei der Schaftadapter einen Schaftansatz (4) mit einer zentralen Bohrung zur Aufnahme des Stiftes (7, 7') mit einem Durchmesser, der im wesentlichen gleich dem des Stiftes ist, und der mit dem Adapterelement (8) in verschiedenen Relativstellungen in Quer- und Längsrichtung gesehen verbindbar ist, aufweist.

3. Kombination nach Anspruch 2, dadurch gekennzeichnet, daß der Schaftansatz (4) auf einer dem Adapterelement (8) abgewandten Seite einen konzentrisch zur Bohrung angeordneten sphärischen Bereich (5) aufweist.

4. Kombination nach Anspruch 3, dadurch gekennzeichnet, daß eine mit dem sphärischen Bereich (5) zusammenwirkende Klemmscheibe (6) vorgesehen ist.

5. Kombination nach einem der Ansprüche 1 bis 4, dadurch gekennzeichnet, daß auf der der aufzunehmenden Stumpffassung (1) abgewandten Seite des Adapterelementes (8) eine eine Bohrung zur Aufnahme des Stiftes aufweisende Scheibe (13) vorgesehen ist, deren Durchmesser größer als die Abmessungen der Ausnehmung (12) ist und der Stift (7, 7') mittels einer auf der der Stumpffassung (1) abgewandten Seite angreifenden Mutter (14) folgt.

6. Kombination nach einem der Ansprüche 1 bis 5, dadurch gekennzeichnet, daß ein zusätzliches Verbindungselement (15) vorgesehen ist, welches auf seiner einen Seite zur Verbindung mit dem Prothesenschaft (21) ausgebildet ist und auf seiner anderen Seite mit dem Adapterelement (8) innerhalb eines um eine Mittenachse des Verbindungselementes (15) gehenden Kegelwinkels schwenkbar verbunden ist.

7. Kombination nach Anspruch 6, dadurch gekennzeichnet, daß das Verbindungselement (15) eine Klemmeinrichtung (20) zum Verbinden mit dem Prothesenschaft (21) aufweist.

## Claims

1. A combination of a shaft adapter for connecting a stump holder to a shaft of a prosthesis and a pin (7, 7') connected to the stump holder (1), the shaft adapter having an adapter element (8) which has a cutout (12) for receiving the pin (7, 7'), and the cross-section of the cutout (12) being greater than the cross-section of the pin (7, 7') such that the pin (7, 7') may be displaced in the cutout (12), and it being possible to fix the pin (7, 7') in any position in the cutout (12), characterized in that, for receiving a bolt (24) connected to a stocking (23) to be received, the pin (7) is constructed as a hollow pin, and a locking device (27, 28, 29) is provided for locking the bolt in a predetermined position in the hollow pin (7).

2. A combination according to Claim 1, the shaft adapter having a shaft shoulder (4) which has a central bore for receiving the pin (7, 7'), the diameter of the bore being substantially the same as that of the pin, and it being possible to connect the shaft shoulder to the adapter element (8) in different relative positions in the transverse and longitudinal direction.

3. A combination according to Claim 2, characterized in that, on a side remote from the adapter element (8), the shaft shoulder (4) has a spherical region (5) arranged concentrically with respect to the bore.

4. A combination according to Claim 3, characterized in that a clamping plate (6) is provided which cooperates with the spherical region (5).

5. A combination according to one of Claims 1 to 4, characterized in that a washer (13) having a bore for receiving the pin is provided on that side of the adapter element (8) which is remote from the stump holder (1) to be received, the diameter thereof being greater than the dimensions of the cutout (12), and the pin (7, 7') follows by means of a nut (14) acting on that side remote from the stump holder (1).

6. A combination according to one of the Claims 1 to 5, characterized in that an additional connecting element (15) is provided which on its one side is constructed to be connected to the shaft (21) of the prosthesis and on its other side is connected to the adapter element (8) such that it is pivotal within a cone angle extending about a centre axis of the connecting element (15).

7. A combination according to Claim 6, characterized in that the connecting element (15) has a clamping device (20) for connection to the shaft (21) of the prosthesis.

## Revendications

1. Système combiné formé par un adaptateur pour tige de prothèse, destiné à assembler une emplanture pour moignon avec la tige d'une prothèse, et par une goupille (7, 7') assemblée avec l'emplanture pour moignon (1), l'adaptateur pour tige de prothèse comportant un organe d'ajustage (8) muni d'un évidement (12) destiné à recevoir la goupille (7, 7') et la section de l'évidement (12) étant supérieure à la section de la goupille (7, 7') de telle sorte que la goupille (7, 7') puisse glisser dans l'évidement (12) et la goupille (7, 7') pouvant être bloquée dans chaque position de l'évidement (12), caractérisé en ce que la goupille (7), destinée à recevoir une cheville (24) assemblée avec un manchon (23) à poser, est conçue sous forme de goupille creuse et il est prévu un dispositif de blocage (27, 28, 29), destiné à bloquer la cheville dans une position prédéfinie dans la goupille creuse (7).

2. Système combiné selon la revendication 1, dans lequel l'adaptateur pour tige de prothèse comprend une rallonge de tige (4), munie d'une forure centrale destinée à recevoir la goupille (7, 7') et présentant un diamètre sensiblement égal à celui de la goupille, et laquelle rallonge de tige peut être assemblée avec l'organe d'ajustage (8) dans diverses positions relatives, par référence au sens transversal et au sens longitudinal.

3. Système combiné selon la revendication 2, caractérisé en ce que la rallonge de tige (4) comprend, sur un côté opposé à l'organe d'ajustage (8), une zone sphérique (5) disposée concentriquement par rapport à la forure.

4. Système combiné selon la revendication 3, caractérisé en ce qu'il est prévu un disque de blocage (6) agissant conjointement avec la zone sphérique (5).

5. Système combiné selon l'une quelconque des revendications 1 à 4, caractérisé en ce que, sur le côté de l'organe d'ajustage (8) opposé à l'emplanture de moignon (1) à recevoir, il est prévu une rondelle (13) munie d'une forure destinée à recevoir la goupille, le diamètre de ladite rondelle est supérieur aux dimensions de l'évidement (12), et la goupille (7, 7') est associée à un écrou (14) qui vient en prise contre le côté opposé à l'emplanture de moignon (1).

6. Système combiné selon l'une quelconque des revendications 1 à 5, caractérisé en ce qu'il est prévu un élément de liaison (15) supplémentaire, dont l'un des côtés est conçu pour l'assemblage avec la tige d'une prothèse (21) et l'autre côté est assemblé avec l'organe d'ajustage (8) à l'intérieur d'un tronc de cône qui entoure l'axe médian de l'élément de liaison (15).

7. Système combiné selon la revendication 6, caractérisé en ce que l'élément de liaison (15) comprend un dispositif de blocage (20) à assembler avec la tige de prothèse (21).
